# EUROPEAN PATENT SPECIFICATION

(11) **EP 1 452 130 B1**
(45) Date of publication and mention of the grant of the patent: **06.08.2008**
(21) Application number: 04003030.6
(22) Date of filing: 11.02.2004
(51) Int. Cl.: A61B 5/00, A61B 5/117, G06F 19/00

(54) **Storage device**
Datenspeicher
Dispositif de stockage de données

(30) Priority: 28.02.2003 JP 2003052410; 30.06.2003 JP 2003186271
(43) Date of publication of application: 01.09.2004
(60) Divisional application: 05002507.1
(73) Proprietor: TANITA CORPORATION, Tokyo (JP)
(72) Inventor: Yoshizawa, Masaki, Tokyo (JP); Miyashita, Yuichi, Tokyo (JP)
(74) Representative: Grünecker, Kinkeldey, Stockmair & Schwanhäusser Anwaltssozietät

(56) References cited:
- EP-A- 0 859 327
- GB-A- 2 377 574
- US-A1- 2002 049 684
- US-A1- 2002 077 077
- US-A1- 2002 147 035
- US-A1- 2002 198 024
- US-A1- 2003 004 397
- US-A1- 2003 034 897
- US-A1- 2003 050 537
- US-B1- 6 454 708
- US-B1- 6 476 825
- US-B1- 6 494 830
- PATENT ABSTRACTS OF JAPAN vol. 2002, no. 06, 4 June 2002 (2002-06-04) & JP 2002 041247 A (NEC CORP), 8 February 2002 (2002-02-08)

## Description

### BACKGROUND OF THE INVENTION

### (i) Field of the Invention

The present invention relates to a biological data acquiring apparatus for acquiring biological data of a user, particularly, to a storage device for a biological data acquiring apparatus and an biological data acquiring apparatus using the storage device.

### (ii) Description of the Related Art

A variety of biological data acquiring apparatuses for acquiring biological data of a user such as a body weight, a body fat percentage, a pulse and the number of steps have been developed and actually employed. These biological data acquiring apparatuses include one which directly measures a body weight as biological data of a user in a manner similar to a scale and one which acquires desired biological data such as a body fat percentage, a body water percentage, a visceral fat level, a muscle mass, a bone density and a basal metabolic rate by performing computations based on a body weight and a bioelectrical impedance value which are measured biological data of a user and biological data such as a height, age and sex which are input by the user (refer to Patent Publications 1 and 2, for example).

Further, in recent years, there are systems that acquire desired biological data that are difficult to acquire by the biological data acquiring apparatuses alone, such as histories of changes in the biological data over a long continuous time period, by transmitting the biological data acquired by the above biological data acquiring apparatuses to a data processing terminal such as a personal computer and storing the data in the terminal or by performing such computations as described above or more complicated computations. As such a system, for example, a system is proposed which acquires biological data such as the number of steps or a waking pace of a user by use of a body motion sensor and a computation unit which are incorporated in the main body of a pedometer, transmits the biological data to a personal computer by use of a communication cable or an optical communication unit, and performs computations based on the biological data and other biological data input in the personal computer such as the height, body weight, age and sex of the user so as to acquire biological data such as a consumed calorie and a history of change thereof (refer to Patent Publication 3).

### Patent Publication 1

Japanese Patent Publication No. 5-49050

### Patent Publication 2

Japanese Patent Publication No. 2001-70273

### Patent Publication 3

Japanese Patent Publication No. 2000-41953

The above conventional biological data acquiring apparatus has the following problems in an attempt to acquire desired biological data by transmitting biological data to a data processing terminal such as a personal computer and storing the biological data in the data processing terminal or performing computations in the data processing terminal.

Firstly, when the biological data acquiring apparatus is connected to the data processing terminal by use of a cable or the like so as to transmit the biological data to the data processing terminal, they must be placed within such an area that the cable can reach both the apparatus and the terminal. Particularly, when both the apparatus and the terminal are generally used in a stationary position (i.e., the stationary devices) as in the case where biological data is transmitted from a scale or such a body fat monitor as disclosed in the above Japanese Patent Publication No. 5-49050 to a so-called desktop type personal computer, a sufficiently long cable must be used or one of the devices must be moved to a position close to the other device upon transmission and reception of the biological data. In general households, a scale and a body fat monitor are often placed in a different room from a room in which a personal computer is set. Thus, using a long cable or moving the devices is not very practical and makes the devices difficult to use. Further, in the case of a biological data acquiring apparatus having a weight sensor such as a scale, an undesired load may be physically applied on the weight sensor depending on the position or state of connection of the cable, thereby degrading the accuracy of measurement. Meanwhile, when one of the biological data acquiring apparatus or the data processing terminal is small and can be carried easily (i.e., portable) as in the case of the pedometer disclosed in the above Japanese Patent Application Laid-Open No. 2000-41953, the possibility of occurrence of the above problem is small. However, in this case as well, handling of a cable is still cumbersome, and it can be hardly said that the devices are easy to use.

Further, when the biological data acquiring apparatus is connected to the data processing terminal in a wireless manner through optical communication such as infrared radiation, communication cannot be made when an object exists between communication units provided to the biological data acquiring apparatus and the data processing terminal. Thus, as in the case of the above connection using a cable, the positions of the devices are limited or the devices must be moved. Accordingly, the wireless connection is also not suitable for the stationary devices. Meanwhile, when an electric wave is used for carrying out the wireless connection, communication is still possible even if an object of a certain size exists, so that the above problems regarding the positions and moving of the devices are nearly completely solved. However, since communication units using electric waves are expensive, the biological data acquiring apparatus and the data processing terminal become expensive. Further, since such communication units consume a large quantity of electric currents, the useful life of a battery used in the biological data acquiring apparatus which is often battery-driven is short, and running costs are high.

US6454708 Discloses a storage device for a biological data acquiring apparatus comprising a connection terminal connectable to the main unit of the biological data acquiring apparatus, biological data storing means and program software stored in the biological data storing means.

The present invention has been conceived by focusing on the above conventional problems. An object of the present invention is to provide a storage device for a biological data acquiring apparatus which makes possible transmission of biological data from the biological data acquiring apparatus to a data processing terminal without use of a cable, infrared radiation, electric wave or the like when the biological data is to be transmitted from the biological data acquiring apparatus to the data processing terminal and computations are to be performed in the data processing terminal based on the biological data so as to eventually acquire desired biological data, and which can be implemented at a relatively low cost.

Another object of the present invention is to provide a biological data acquiring apparatus which can transmit biological data to a data processing terminal without use of a cable, infrared radiation, electric wave or the like when the biological data is to be transmitted from the biological data acquiring apparatus to the data processing terminal and computations are to be performed in the data processing terminal based on the biological data so as to eventually acquire desired biological data, and which can be implemented at a relatively low cost.

### SUMMARY OF THE INVENTION

A storage device according to claim 1 for a biological data acquiring apparatus is provided, which is independent and detachable from the main unit of the biological data acquiring apparatus.

Alternatively, a biological data acquiring apparatus according to the present invention comprises a main unit incorporating biological data acquiring means for acquiring biological data and a storage device according to claim 1.

preferred embodiments are set out in the dependent claims.

### BRIEF DESCRIPTION OF THE DRAWINGS

Fig. 1 is an external view of a storage device for a biological data acquiring apparatus according to the present invention.
Fig. 2 is an external view of the biological data acquiring apparatus according to the present invention.
Fig. 3 is a schematic diagram showing electronic circuit boards incorporated in the biological data acquiring apparatus according to the present invention.
Fig. 4 is a flowchart showing a flow in using the biological data acquiring apparatus according to the present invention.
Fig. 5A is a flowchart showing the data storing operation of the storage device according to the present invention.
Fig. 5B is a flowchart showing the data transmitting operation of the storage device according to the present invention.

### DETAILED DESCRIPTION OF THE PREFERRED EMBODIMENTS

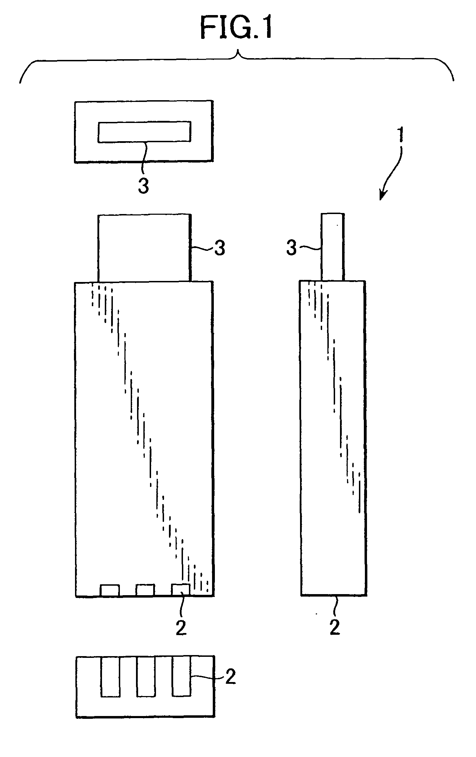

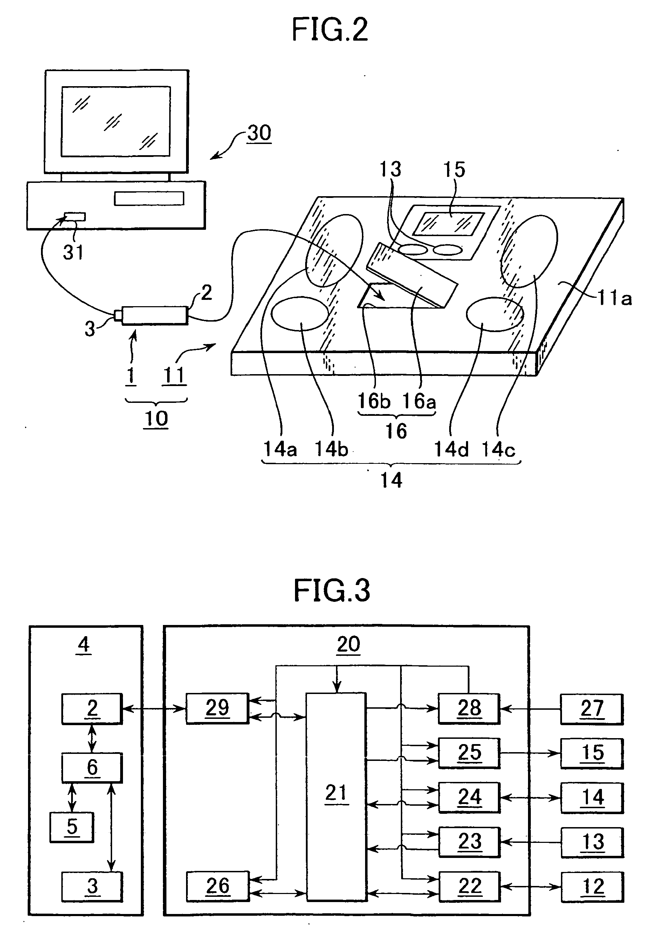

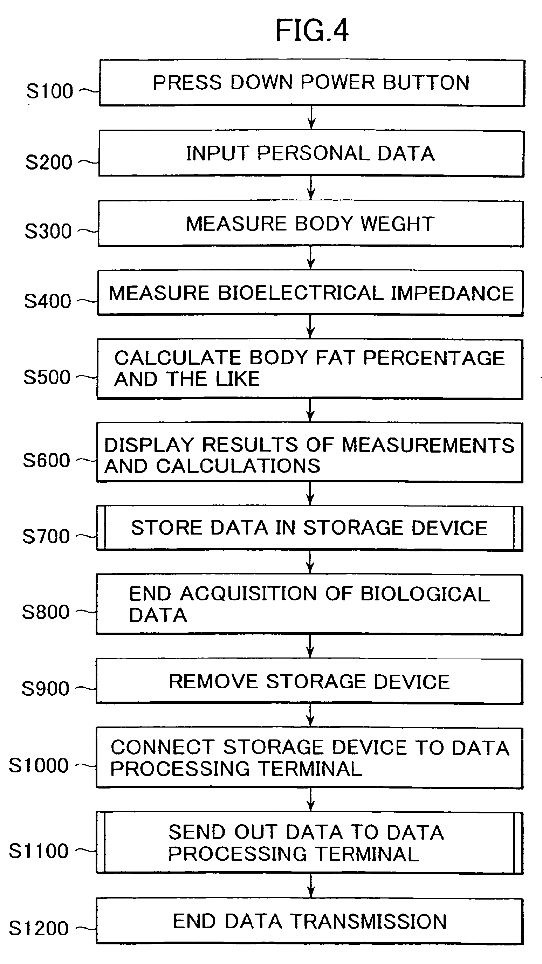

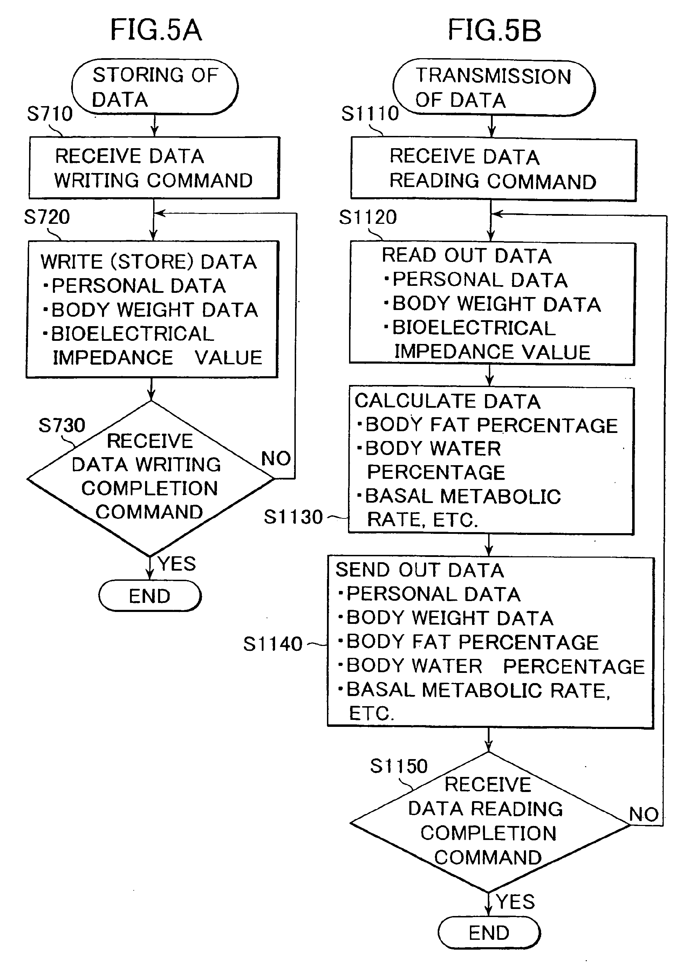

Hereinafter, a preferred embodiment of the present invention will be described with referring to the drawings. In Fig. 1, a storage device 1 for a biological data acquiring apparatus according to the present invention is shown. In Fig. 2, together with a data processing terminal 30, a biological data acquiring apparatus 10 according to the present invention which uses the storage device 1 is shown. Further, Fig. 3 is a schematic diagram showing the constitutions of electronic circuit boards incorporated in the biological data acquiring apparatus 10. Figs. 4 and 5 are flowcharts showing steps in acquiring biological data by use of the biological data acquiring apparatus 10 and sending the acquired biological data to the data processing terminal 30.

The storage device 1 is an improved version of a so-called USB memory which has already been commercially available in various forms as an external memory for a personal computer having a terminal which meets the universal serial bus (USB) standard. The storage device 1 has a size and a shape which are nearly the same as those of a chewing gum package. As shown in Fig. 1, it has a connection terminal 2 for the main unit 11 of the biological data acquiring apparatus 10, at one end in its longitudinal direction, and has a connection terminal 3 for the data processing terminal 30, at the other end. Of the two, the connection terminal 3 is a male USB terminal and can be easily connected to a variety of data processing terminals having female USB terminals. As a matter of course, the connection terminal 3 may also be a female USB terminal or a terminal of different standard and shape from the USB standard.

The main unit 11 of the biological data acquiring apparatus 10 is an improved version of a so-called scale with a body fat monitor which has already been commercially available in various forms as a stationary apparatus which calculates a body fat percentage, a body water percentage, a visceral fat level, a muscle mass, a bone density, a basal metabolic rate and the like (hereinafter generically and simply referred to as "body fat percentage" in the present embodiment) by measuring the body weight and biological impedance value of a user. As shown in Fig. 2, its top surface 11a serves as a scale platform for measuring a body weight which uses a straining member (i.e., loadcell) 12 (refer to Fig. 3). On the top surface 11a, there are provided operation switches 13 which are used by a user to enter a date and time of measurement and biological data (hereinafter generically referred to as "personal data" in the present embodiment) such as a height, age and sex, electrodes 14 which make contact with the bottoms of the feet of a user so as to measure a biological impedance value, a liquid crystal display 15 for displaying the results of measurements and calculations, and a mounting section 16 to which the storage device 1 is attached.

The electrodes 14 comprise electrodes 14a and 14b which make contact with the bottom of the left foot of a user and electrodes 14c and 14d which make contact with the bottom of the right foot of the user. Of these electrodes, a feeble electric current can be passed between both feet by use of the electrodes 14a and 14c which make contact with the toe sides of both feet, and an impedance value between both feet can be measured by use of the electrodes 14b and 14d which make contact with the heel sides of both feet. Nearly at the center of a portion sandwiched by the electrodes 14a and 14b for the left foot and the electrodes 14c and 14d for the right foot on the top surface 11a of the main unit, the mounting section 16 for the storage device 1 is formed. The mounting section 16 is formed as a cavity 16b having an openable/closable cover 16a with a sufficient size in which the storage device 1 can be set in a laid position. On the internal surface of the cavity 16b, there is provided a connection terminal 29 (refer to Fig. 3) which corresponds to the connection terminal 2 of the storage device 1.

When the mounting section 16 for the storage device 1 is formed on the top surface 11a of the main unit 11 as described above, the storage device 1 can be attached and removed more easily than when the mounting section 16 is formed on the side or rear surface of the main unit 11. Further, when the mounting section 16 has the foregoing structure that the storage device 1 can be set therein in a laid position, the storage device 1 can be prevented from sticking out from the top surface 11a at the time of its attachment without increasing the thickness of the main unit 11 so much, thereby making it difficult to occur that a user mistakenly steps on the storage device 1 and breaks it. Further, with the openable/closable cover 16a, the possibility of occurrence of such a breakage is further reduced to a significant degree. As a matter of course, the position and shape of the mounting section 16 are not limited to those in the present embodiment and may be any appropriate position and shape according to the sizes and other features of the storage device 1 and main unit 11. For example, a biological data acquiring apparatus is in existence, into which the operation switches 13 and the liquid crystal display 15 have been installed as units detachable or independent from the main unit 11. In the case of such a biological data acquiring apparatus, the mounting section 16 may be formed in the unit.

The biological data acquiring apparatus 10 (storage device 1 and main unit 11) incorporates electronic circuit boards having the constitutions shown in Fig. 3. Aboard 4 which is incorporated in the storage device 1 has the connection terminal 2, the connection terminal 3, a flash memory 5 as biological data storing means, and a microprocessor 6. The microprocessor 6 comprises a ROM which stores program software for writing biological data in the flash memory 5, reading biological data from the flash memory 5 and performing calculations of the biological data, a CPU and a RAM as operation elements for executing the program software, and other elements. The storage device 1 is supplied with electric power from the main unit 11 when connected to the main unit 11 or from the data processing terminal 30 when connected to the data processing terminal 30. It is needless to say that the biological data storing means may be any rewritable storage medium capable of holding data. Therefore, for example, an EEPROM can be used in place of the flash memory 5.

Meanwhile, a board 20 which is incorporated in the main unit 11 has a microprocessor 21 which comprises a CPU, a ROM and a RAM for measuring and calculating the body weight and body fat percentage of a user. To the microprocessor 21, a scale circuit 22 which is connected to the straining member 12 for measuring a body weight, an input circuit 23 which is connected to the operation switches 13 for inputting personal data, an impedance circuit 24 which is connected to the electrodes 14 for measuring a bioelectrical impedance, a display circuit 25 which is connected to the liquid crystal display 15 for displaying measured or calculated biological data such as a body weight and a body fat percentage, an EEPROM 26 which stores measured or calculated biological data, an electric power source circuit 28 which is connected to a battery 27 for driving the biological data acquiring apparatus 10, and a connection terminal 29 which corresponds to the connection terminal 2 of the storage device 1 are connected. The microprocessor 21, the straining member 12 and the scale circuit 22, the operation switches 13 and the input circuit 23, the electrodes 14 and the impedance circuit 24, and other components all together constitute biological data acquiring means incorporated in the main unit 11.

Meanwhile, the data processing terminal 30 shown in Fig. 2 is a so-called desktop personal computer in the present embodiment. It has an USB-standard-based connection terminal 31 for connecting to an external peripheral device and can be easily connected with the connection terminal 3 of the storage device 1 accordingly. In the data processing terminal 30, a program software for not only accumulating various data acquired by the biological data acquiring apparatus 10 such as personal data, body weight data and a body fat percentage continuously but also displaying histories of changes of the data as graphs on a monitor (hereinafter referred to as "history management software" in the present embodiment) is installed. As a matter of course, program software to be installed in the data processing terminal 30 is not limited to such history management software. For instance, software which gives a user an advice regarding health management based on the data acquired by the biological data acquiring apparatus 10 such as a body weight and a body fat percentage and software which sends these data to others (such as a personal doctor) via the Internet are conceivable.

Next, a flow of operations in acquiring data such as personal data, body weight data and a body fat percentage by use of the biological data acquiring apparatus 10 and sending the acquired data to the data processing terminal will be described.

In the flowchart of Fig. 4, firstly, when a user presses down a power button (not shown) on the main unit 11 (S100), an electric current from the battery 27 is fed to the circuits and the microprocessor 21 on the board 20 via the electric power source circuit 28. At this time, if the storage device 1 is set in the mounting section 16 and the connection terminal 2 is connected to the connection terminal 29, the electric current is also supplied to the storage device 1 via the connection terminals 29 and 2.

Then, the user operates the operation switches 13 so as to input personal data (S200). The personal data is sent to the microprocessor 21 via the input circuit 23 and stored in the RAM of the microprocessor 21 temporarily. It is also possible that the personal data is stored in the EEPROM 26 together with a personal number set for each user and the user causes the microprocessor 21 to retrieve the personal data from the EEPROM 26 simply by entering the personal number through the operation switches 13 on the next measurement.

Then, when the user stands on the main unit 11 with the bottoms of both feet in contact with the electrodes 14, the strain of the straining member 12 is input into the microprocessor 21 via the scale circuit 22 so as to measure a body weight (S300). At the same time, an electric current value detected by the electrodes 14 is input into the microprocessor 21 via the impedance circuit 24 so as to measure a bioelectrical impedance value (S400).

Then, the CPU in the microprocessor 21 executes program software stored in the ROM so as to calculate the body fat percentage of the user based on the personal data input in S200, the body weight data measured in S300 and the bioelectrical impedance value measured in S400 (S500). That is, this program software includes a regression formula for calculating a body fat percentage based on the personal data, the body weight data and the bioelectrical impedance value (hereinafter referred to as "regression operation software" in the present example).

Then, the microprocessor 21 displays the body weight data measured in S300 and the data such as the body fat percentage calculated in S500 on the liquid crystal display 15 via the display circuit 25 (S600). At this time, if the storage device 1 is set in the mounting section 16 and the connection terminal 2 is connected to the connection terminal 29, predetermined biological data to be described later is stored in the flash memory 5 as the biological data storing means which is incorporated in the storage device 1 (S700).

Hereinafter, the storing operation in S700 will be described in accordance with the flowchart in Fig. 5A in addition to Fig. 4. Firstly, after completion of S600 shown in Fig. 4, the microprocessor 21 of the main unit 11 sends a data writing command to the storage device 1 and also starts transmission of biological data to be described later. Upon receipt of the data writing command (S710), the microprocessor 6 of the storage device 1 writes and stores the biological data sent from the main unit 11 in the flash memory 5 sequentially (S720). The biological data sent from the main unit 11 to the storage device 1 and stored in the flash memory 5 are the personal data input in S200, the body weight data measured in S300 and the bioelectrical impedance value measured in S400. The data of the body fat percentage calculated in S500 is however not stored in the flash memory 5 so as to reduce the capacity of the flash memory 5.

Then, when a data writing completion command is sent from the microprocessor 21 of the main unit 11 to the microprocessor 6 of the storage device 1 (S730) after completion of writing of the biological data to the flash memory 5, the operation of storing the biological data into the flash memory 5 in S700 of Fig. 4 is completed. Thereby, the operation of acquiring the personal data, the body weight data and the data of the body fat percentage by use of the biological data acquiring apparatus 10 is completed (S800).

Then, in order to transfer the personal data, the body weight data and the data of the body fat percentage acquired by the biological data acquiring apparatus 10 to the data processing terminal 30, the user removes, from the main unit 11, the storage device 1 which is independent and detachable from the main unit 11 (S900), carries the storage device 1 to where the data processing terminal 30 is placed, and connects the connection terminal 3 to the connection terminal 31 of the data processing terminal 30 (S1000). Then, for example, when the history management software installed in the data processing terminal 30 is activated, the personal data, the body weight data and the data of the body fat percentage are sent out from the storage device 1 to the data processing terminal 30 (S1100).

Hereinafter, the data sending operation in S1100 will be described in accordance with the flowchart of Fig. 5B in addition to Fig. 4. Firstly, in accordance with the history management software, the data processing terminal 30 sends a data reading command to the storage device 1. Upon receipt of the data reading command (S1110), the microprocessor 6 of the storage device 1 reads out the personal data, body weight data and bioelectrical impedance value stored in the flash memory 5 (S1120) and executes program software stored in the ROM so as to calculate the body fat percentage (S1130). The program software executed above is the same as the regression operation software stored in the microprocessor 21 of the main unit 11. Then, the personal data, the body weight data and the data of the body fat percentage are sent from the connection terminal 3 to the data processing terminal 30 (S1140).

Thereafter, when a data reading completion command is sent from the data processing terminal 30 to the microprocessor 6 of the storage device 1 (S1150) after completion of receipt of the personal data, the body weight data and the data of the body fat percentage in the data processing terminal 30, the data sending operation in S1100 of Fig. 4 is completed. Thereby, the operation of sending out the personal data, the body weight data and the data of the body fat percentage by use of the storage device 1 of the biological data acquiring apparatus 10 is completed (S1200).

As described above, the storage device 1 of the biological data acquiring apparatus 10 in the present embodiment is independent and detachable from the main unit 11 and has the flash memory 5 for storing the personal data, body weight data and bioelectrical impedance value which are acquired by means of the straining member 12, the operation switches 13, the electrodes 14 and the microprocessor 21, those being incorporated in the main unit 11. Thus, the storage device 1 can be removed from the main unit 11 with the personal data, body weight data and bioelectrical impedance value stored in the flash memory 5. Further, the storage device 1 can be connected to the data processing terminal 30 so as to send the personal data and body weight data stored in the flash memory 5 to the data processing terminal 30.

In other words, the biological data acquiring apparatus 10 in the present embodiment comprises the main unit 11 and the storage device 1; the main unit incorporating the straining member 12, the operation switches 13, the electrodes 14 and the microprocessor 21, for acquiring the personal data, the body weight data and the data of the body fat percentage; while, the storage device 1 being independent and detachable from the main unit 11 and having the flash memory 5 for storing the personal data, body weight data and bioelectrical impedance value which are acquired by the main unit 11. The biological data acquiring apparatus 10 therefore enables the storage device 1 to be removed from the main unit 11 with the personal data, body weight data and bioelectrical impedance value stored in the flash memory 5. Further, the storage device 1 can be connected to the data processing terminal 30 so as to send the personal data and body weight data stored in the flash memory 5 to the data processing terminal 30.

Further, the storage device 1 has the microprocessor 6 which comprises the ROM containing the regression operation software for calculating the body fat percentage based on the personal data, body weight data and bioelectrical impedance value stored in the flash memory 5, and the CPU and RAM for executing the regression operation software. Thus, calculation of the body fat percentage can be performed in the storage device 1, and then the data of the calculated body fat percentage can be sent to the data processing terminal 30. As a result, there is no need to store all the data of the body fat percentage in the flash memory 5, and a flash memory 5 with a small capacity is used to make the storage device 1 or the biological data acquiring apparatus 10 inexpensive.

Further, although the main unit 11 is an improved version of a stationary scale with a body fat monitor, there is no need to move the main unit 11 when the personal data, the body weight data and the data of the body fat percentage are sent to the data processing terminal 30, thereby making the biological data acquiring apparatus 10 easy to use.

In the present embodiment, the microprocessor 6 which stores and further executes the regression operation software is included in the storage device 1. However, it is also possible that the storage device 1 merely stores such regression operation software and the data processing terminal 30 actually executes the software. In this case, the storage device 1 is connected to the data processing terminal 30 so as to send the personal data, body weight data and bioelectrical impedance value thereto and subsequently the data processing terminal 30 accesses and executes the regression operation software stored in the storage device 1 by utilizing the data and value, so as to receive the data of the calculated body fat percentage from the storage device 1. With such a constitution as well, there is no need to store all the data of the body fat percentage in the flash memory 5, so that a flash memory 5 with a small capacity can be used to make the storage device 1 and the biological data acquiring apparatus 10 inexpensive.

In addition, the constitutions of the storage device and biological data acquiring apparatus to which the present invention is applied do not have to be limited to descriptions in the present embodiment, and various modifications can be made on the constitutions, as has been described accordingly together with the description of the embodiment. Further, the present invention is not limited to an apparatus which measures bioelectrical impedance between the bottoms of both feet of a user as in the embodiment and can also be widely applied to apparatuses which measure bioelectrical impedance between both hands, between a hand and a foot or between other specific body parts of a user. Further, the present invention is not limited to a biological data acquiring apparatus of the type which measures the body weight or bioelectrical impedance of a user as in the present embodiment and can also be widely applied, for example, to an apparatus which measures or calculates a thickness of fat or a bone density by use of the ultrasonic wave or the like as well as to various known apparatuses for acquiring biological data, e.g., a blood pressure meter, a pulse meter, a pedometer and a consumed calorie meter. Further, it is needless to say that the present invention can be widely applied to portable biological data acquiring apparatuses, in addition to stationary biological data acquiring apparatuses.

As described above, the storage device for a biological data acquiring apparatus according to the present invention is independent and detachable from the main unit of the biological data acquiring apparatus that incorporates biological data acquiring means for acquiring biological data and has biological data storing means for storing the biological data acquired by the biological data acquiring means. Thus, the storage device can be detached from the main unit of the biological data acquiring apparatus with the biological data stored in the biological data storing means and can be connected to a data processing terminal so as to transmit the stored biological data to the data processing terminal. Further, by accumulating the biological data in the data processing terminal or performing computations in the data processing terminal, desired biological data which are difficult to acquire by the biological data acquiring apparatus alone can be acquired. Especially, since the above storage device has program software for performing computations based on the biological data stored in the above biological data storing means, the program software can be executed in the data processing terminal when the storage device is connected to the data processing terminal. As a result, the need to store all biological data in the biological data storing means is obviated, and the storage capacity of the biological data storing means can be kept small, thereby making the storage device relatively inexpensive.

Further, when the above storage device has an operation element for executing the program software, the program software can be executed in the storage device, and biological data resulting from the execution of the software can be transmitted to the data processing terminal. As a result, the need to store all biological data in the biological data storing means is obviated, and the storage capacity of the biological data storing means can be kept small, thereby making the storage device relatively inexpensive.

Further, even when the above main unit of the biological data acquiring apparatus is stationary, the usability of the biological data acquiring apparatus can be improved because there is no need to move the main unit in transmitting biological data to the data processing terminal.

Alternatively, the biological data acquiring apparatus according to the present invention comprises a main unit which incorporates biological data acquiring means for acquiring biological data and a storage device which is independent and detachable from the main unit and has biological data storing means for storing the biological data acquired by the above biological data acquiring means. Thus, the storage device can be detached from the main unit with the biological data stored in the biological data storing means and can be connected to a data processing terminal so as to transmit the stored biological data to the data processing terminal. Further, by accumulating the biological data in the data processing terminal or performing computations in the data processing terminal, desired biological data which are difficult to acquire by the biological data acquiring apparatus alone can be acquired. Especially, since the above storage device has program software for performing computations based on the biological data stored in the above biological data storing means, the program software can be executed in the data processing terminal when the storage device is connected to the data processing terminal. As a result, the need to store all biological data in the biological data storing means is obviated, and the storage capacity of the biological data storing means can be kept small, thereby making the biological data acquiring apparatus relatively inexpensive.

Further, when the above storage device has an operation element for executing the program software, the program software can be executed in the storage device, and biological data resulting from the execution of the software can be transmitted to the data processing terminal. As a result, the need to store all biological data in the biological data storing means is obviated, and the storage capacity of the biological data storing means can be kept small, thereby making the biological data acquiring apparatus relatively inexpensive.

Further, even when the above main unit is stationary, the biological data acquiring apparatus can be of good usability because there is no need to move the main unit in transmitting biological data to the data processing terminal.

## Claims

1. A storage device (1) for a biological data acquiring apparatus (10) that incorporates biological data acquiring means (12, 13, 14, 21, 22, 23, 24) for acquiring biological data, wherein the storage device (1) is independent and detachable from a main unit (11) of the biological data acquiring apparatus (10) and comprises:
a first connection terminal (2) connectable to the main unit (11) of the biological data acquiring apparatus (10) for sending the biological data from the main unit (11) of the biological data acquiring apparatus (10) to the storage device (1), wherein the storage device (1) is supplied with electrical power from the main unit (11) when connected to the main unit (11),
biological data storing means (5) for storing the biological data acquired by the biological data acquiring means (12, 13, 14, 21, 22, 23, 24), and
program software for performing computations based on the biological data stored in the biological data storing means (5), **characterised in that** it comprises
a second connection terminal (3), which is different from the first connection terminal and connectable to a data processing terminal (30) for sending the biological data from the storage device (1) to a data processing terminal (30), wherein the storage device (1) is supplied with electrical power from the data processing terminal (30) when connected to the data processing terminal (30).

2. The storage device (1) of claim 1, further having an operation element (6) for executing the program software.

3. The storage device (1) of claim 1 or 2, wherein the first connection terminal (2) connectable to the main unit (11) of the biological data acquiring apparatus (10) and the second connection terminal (3) connectable to the data processing terminal (30) are combined into one connection terminal connectable to the main unit (11) of the biological data acquiring apparatus (10) and the data processing terminal (30).

4. A biological data acquiring apparatus (10) comprising:
a main unit (11) incorporating biological data acquiring means (12, 13, 14, 21, 22, 23, 24) for acquiring biological data and
a storage device (1) according to any of the claims 1 to 3.

5. The apparatus of claim 4, wherein the main unit (11) of the biological data acquiring apparatus (10) is stationary.

## Patentansprüche

1. Ein Speichermedium (1) für eine biologische Daten empfangende Vorrichtung (10), die biologische Daten empfangende Mittel (12, 13, 14, 21, 22, 23, 24) zum Empfang biologischer Daten einschließt, wobei das Speichermedium (1) selbstständig und abtrennbar von der Haupteinheit (11) der biologische Daten empfangenden Vorrichtung (10) ist und das Speichermedium (1) umfasst:
einen ersten Anschlussbereich (2), der mit der Haupteinheit (11) der biologische Daten empfangenden Vorrichtung (10) verbindbar ist zum Senden von biologischen Daten von der Haupteinheit (11) der biologische Daten empfangenden Vorrichtung (10) zu dem Speichermedium (1), wobei das Speichermedium (1) mit elektrischem Strom aus der Haupteinheit (11) versorgt wird, sobald es mit der Haupteinheit (11) verbunden ist;
biologische Daten speichernde Mittel (5) zum Speichern von biologischen Daten, die von den biologische Daten empfangenden Mitteln (12, 13, 14, 21, 22, 23, 24) empfangen werden; und
Programm-Software zum Ausführen von Berechnungen, die auf biologischen Daten basieren, welche in den biologische Daten speichernden Mittel (5) gespeichert sind;
das Speichermedium (1) charakterisiert durch
einen zweiten Anschlussbereich (3), der sich von dem ersten Anschlussbereich (2) unterscheidet und mit dem Datenverarbeitungsterminal (30) verbindbar ist, um biologische Daten von dem Speichermedium (1) zu dem Datenverarbeitungsterminal (30) zu senden, wobei das Speichermedium (1) mit elektrischem Strom aus dem Datenverarbeitungsterminal (30) versorgt wird, sobald es mit dem Datenverarbeitungsterminal (30) verbunden ist.

2. Das Speichermedium nach Anspruch 1, zusätzlich umfassend ein Funktionselement (6) zum Ausführen der Programm-Software.

3. Das Speichermedium nach Anspruch 1 oder 2, wobei der erste Anschlussbereich (2) verbindbar mit der Haupteinheit (11) der biologische Daten empfangenden Vorrichtung (10) und der zweite Anschlussbereich (3) verbindbar mit dem Datenverarbeitungsterminal (30) zu einem Anschlussbereich vereinbar sind, der mit der Haupteinheit (11) der biologische Daten empfangenden Vorrichtung (10) und dem Datenverarbeitungsterminal (30) verbindbar ist.

4. Eine biologische Daten empfangende Vorrichtung (10) umfassend:
eine Haupteinheit (11), die biologische Daten empfangende Mittel (12, 13, 14, 21, 22, 23, 24) zum Empfangen biologischer Daten einschließt;
ein Speichermedium (1) nach einem der Ansprüche 1 bis 3.

5. Die Vorrichtung nach Anspruch 4, wobei die Haupteinheit (11) der biologische Daten empfangenden Vorrichtung (10) ortsfest ist.

## Revendications

1. Dispositif de stockage (1) destiné à un appareil d'acquisition de données biologiques (10) qui comprend un moyen d'acquisition de données biologiques (12, 13, 14, 21, 22, 23, 24) destiné à acquérir des données biologiques, dans lequel le dispositif de stockage (1) est indépendant et peut être détaché d'une unité principale (11) de l'appareil d'acquisition de données biologiques (10) et comprend :
une première borne de raccordement (2) pouvant être reliée à l'unité principale (11) de l'appareil d'acquisition de données biologiques (10) afin d'envoyer les données biologiques entre l'unité principale (11) de l'appareil d'acquisition de données biologiques (10) et le dispositif de stockage (1), dans lequel le dispositif de stockage (1) est alimenté avec une énergie électrique provenant de l'unité principale (11) lorsqu'il est relié à l'unité principale (11),
un moyen de stockage de données biologiques (5) destiné à stocker les données biologiques acquises par le moyen d'acquisition de données biologiques (12, 13, 14, 21, 22, 23, 24), et
un logiciel de programme destiné à effectuer des calculs sur la base des données biologiques stockées dans le moyen de stockage de données biologiques (5), **caractérisé en ce qu'**il comprend une seconde borne de raccordement (3), qui est différente de la première borne de raccordement et qui peut être reliée à un terminal de traitement de données (30) afin d'envoyer les données biologiques entre le dispositif de stockage (1) et un terminal de traitement de données (30), dans lequel le dispositif de stockage (1) est alimenté avec une énergie électrique provenant du terminal de traitement de données (30) lorsqu'il est relié au terminal de traitement de données (30).

2. Dispositif de stockage (1) selon la revendication 1, ayant en outre un élément opérationnel (6) destiné à exécuter le logiciel de programme.

3. Dispositif de stockage (1) selon la revendication 1 ou 2, dans lequel la première borne de raccordement (2) pouvant être reliée à l'unité principale (11) de l'appareil d'acquisition de données biologiques (10) et la seconde borne de raccordement (3) pouvant être reliée au terminal de traitement de données (30) sont combinées en une seule borne de raccordement pouvant être reliée à l'unité principale (11) de l'appareil d'acquisition de données biologiques (10) et au terminal de traitement de données (30).

4. Appareil d'acquisition de données biologiques (10) comprenant :
une unité principale (11) comprenant un moyen d'acquisition de données biologiques (12, 13, 14, 21, 22, 23, 24) destiné à acquérir des données biologiques, et
un dispositif de stockage (1) selon l'une quelconque des revendications 1 à 3.

5. Appareil selon la revendication 4, dans lequel l'unité principale (11) de l'appareil d'acquisition de données biologiques (10) est stationnaire.
